# EUROPÄISCHE PATENTSCHRIFT

(11) **EP 3 125 722 B1**
(45) Veröffentlichungstag und Bekanntmachung des Hinweises auf die Patenterteilung: **02.03.2022**
(21) Anmeldenummer: 15713144.2
(22) Anmeldetag: 19.03.2015
(51) Int. Cl.: A46B 15/00, A61C 17/06, A61B 17/24, A46B 17/08

(54) **ZUNGENREINIGER**
TONGUE CLEANER
GRATTE-LANGUE

(30) Priorität: 20.03.2014 DE 202014002456 U; 28.01.2015 DE 202015000686 U
(43) Veröffentlichungstag der Anmeldung: 08.02.2017
(73) Patentinhaber: TSPRO GmbH, 76227 Karlsruhe (DE)
(72) Erfinder: GEORGI, Matthias, 77886 Lauf (DE); GEIBERGER, Christoph, 53797 Lohmar (DE)
(74) Vertreter: Grünecker Patent- und Rechtsanwälte PartG mbB
(86) Internationale Anmeldenummer: PCT/EP2015/055770
(87) Internationale Veröffentlichungsnummer: WO 2015/140247

(56) Entgegenhaltungen:
- WO-A1-98/42264
- WO-A1-2006/074911
- US-A- 1 983 601
- US-A- 5 984 935

## Beschreibung

Die vorliegende Erfindung betrifft einen Zungenreiniger.

Zungenreiniger für die menschliche Mundhygiene sind heutzutage allgemein in Benutzung. Sie sind u.a. auf der Rückseite von Zahnbürsten ausgebildet, üblicherweise in Form von Noppen, die aus einem weichelastischen Kunststoff, üblicherweise TPE, als Umspritzung auf der Rückseite des Zahnbürsten-Grundkörpers aus PP vorgesehen sind. Damit wird die Idee verfolgt, gleichzeitig zu dem Putzen der Zähne auch die Rückseite der Zahnbürste an der Zunge zur Einwirkung zu bringen, um dort anhaftende Verschmutzung, wie beispielsweise abgestorbene Zellen oder Speisereste abzuschaben und im Rahmen des Zähneputzens aus dem Mundraum zu entfernen. Auch sind Zungenreiniger bekannt, die lediglich als solches wirken, d.h. kein Borstenfeld einer Zahnbürste aufweisen. Diese sind in etwa wie eine Handzahnbürste gestaltet und haben an ihrem nutzungsseitigen Ende ein mit Noppen versehenes Kissen, welches auf einem Kopf angeordnet ist, der Teil eines Grundkörpers des Zungenreinigers ist.

WO 98/42264 A1 offenbart einen Zungenreiniger mit einem im Wesentlichen scheibenförmigen Körper.

Die vorliegende Erfindung will einen verbesserten Zungenreiniger angeben. Dabei will die vorliegende Erfindung insbesondere einen Zungenreiniger angeben, der eine verbesserte Putzwirkung zeigt.

Zur Lösung dieses Problems wird der vorliegenden Erfindung ein Zungenreiniger mit den Merkmalen von Anspruch 1 angegeben. Dieser Zungenreiniger hat einen im Wesentlichen scheibenförmigen Körper. Der scheibenförmige Körper zeichnet sich dadurch aus, dass er eine nutzungsseitige, im Wesentlichen scheibenförmige Vorderfläche hat. Die Vorderfläche hat eine im Wesentlichen scheibenförmige, d.h. ebene Erstreckung, um möglichst flächig gegen die Oberfläche der Zunge angelegt werden zu können. Auf der Vorderfläche ist bei dem erfindungsgemäßen Zungenreiniger eine Profilierung vorgesehen. Diese Profilierung ist zum Abschaben von oberflächlich an der menschlichen Zunge anhaftender Verschmutzung geeignet ausgeformt. Dazu hat die Profilierung üblicherweise Noppen und/oder Rippen, durch welche besagte Verschmutzung abgeschabt werden kann. Zur Verwirklichung der Profilierung des erfindungsgemäßen Zungenreinigers können sämtliche, insbesondere in Verbindung mit Zungenreinigern bei Zahnbürsten bekannte Ausgestaltungen zur Anwendung kommen. Der Körper ist ferner mit einem Schlauchanschluss versehen. Dieser Schlauchanschluss befindet sich üblicherweise unterhalb, bestenfalls auf Höhe der nutzungsseitigen Vorderfläche. Insbesondere überragt der Schlauchanschluss üblicherweise die Vorderfläche nicht, so dass eine schonende Reinigung der menschlichen Zunge möglich ist. Der Schlauchanschluss geht regelmäßig absatzfrei in die Vorderfläche über und vorzugsweise seitlich davon ab, d.h. erstreckt sich mit seiner Längsachse parallel zu einer Ebene, in der im Wesentlichen die Vorderfläche liegt. Der Schlauchanschluss ist derart vorgesehen, dass er mit der Profilierung kommuniziert. Hierzu sind in dem Körper ein oder mehrere Kanäle vorgesehen, durch welche üblicherweise innerhalb der Profilierung vorgesehene Lüftungsöffnungen mit dem Schlauchanschluss kommunizieren. Über einen an den Schlauchanschluss angeschlossenen Absaugschlauch kann dementsprechend beim Einwirken des Zungenreinigers gelöste Verschmutzung durch das Absaugen abgefördert werden.

Durch diese Ausgestaltung wird eine verbesserte Putzwirkung geschaffen. Der erfindungsgemäße Zungenreiniger eignet sich nicht nur durch seine Profilierung dazu, Verschmutzung von der Zunge zu lösen. Vielmehr kann über einen an dem Schlauchanschluss angeschlossenen Absaugschlauch die so gelöste Verunreinigung zielgerichtet und effektiv aus dem Mundraum abgefördert werden.

Dabei ist insbesondere an den Einsatz des erfindungsgemäßen Zungenreinigers in der professionellen Dentalpflege gedacht. Bei der professionellen Dentalpflege bzw. -hygiene wird der erfindungsgemäße Zungenreiniger über den Schlauchanschluss an einen Absaugschlauch angeschlossen, der üblicherweise ohnehin als Unterdruckquelle bei der zahnärztlichen Behandlung bzw. Prophylaxe zur Verfügung steht.

Der Zungenreiniger ist üblicherweise ein Einweg-Teil. Er kann als Wegwerf-Teil ausgebildet sein. Alternativ ist es auch möglich, den Zungenreiniger zu desinfizieren. Vorzugsweise ist der Zungenreiniger als einfaches, einteiliges Spritzgussteil hergestellt. Mit anderen Worten werden sämtliche, nachstehend noch näher erläuterten Bestandteile des Zungenreinigers durch einen einheitlichen Spritzgusskörper gebildet. Zur Herstellung des Zungenreinigers reicht die Verwendung einer einzigen Kunststoffkomponente aus. Dabei kann es sich um eine Hartkomponente oder um eine Weichkomponente handeln. Vorzugsweise kann der Zungenreiniger auch mittels Mehrkomponenten-Spritzgießen hergestellt sein. So kann an einem PP- oder PE- bzw. PA-Grundkörper eine Umspritzung aus einem weichelastischen Kunststoff angespritzt sein. Dieser weichelastische Kunststoff , beispielsweise TPE, kann ganz oder teilweise die Profilierung ausbilden. Er kann auch eine Dichtung für den Anschluss eines Absaugschlauches an den Schlauchanschluss ausformen. Darüber hinaus kann die weichelastische Komponente auch solche Bereiche des Grundkörpers umhüllen, die im Rahmen der üblichen Benutzung am ehesten gegen die empfindlichen Mundschleimhäute stoßen, um diese zu schützen.

Mit Blick auf die beengten Platzverhältnisse im menschlichen Mundraum ist der Körper möglichst flach ausgebildet. Vorzugsweise ist der Körper im Wesentlichen tellerförmig ausgebildet und mit einem Zylinderstutzen versehen, der den Schlauchanschluss ausformt. Dieser Zylinderstutzen überragt vorzugsweise rückseitig den im Wesentlichen tellerförmigen, d.h. flachen Körper des Zungenreinigers. Der Zylinderstutzen ist im Wesentlichen flächenbündig mit der nutzungsseitigen Vorderfläche des Körpers angeordnet. Vorzugsweise geht der Zylinderstutzen im Wesentlichen absatzfrei in einen umlaufenden Rand über, der den tellerförmigen Körper umfänglich umgibt.

Der Schlauchanschluss kommuniziert vorzugsweise mit einem oder mehreren Kanälen innerhalb des Körpers, der bzw. die die an dem Zungenreiniger angeschlossene Saugleitung in dem Körper des Zungenreinigers fortsetzen und zu der Profilierung weiterführen. Mit Blick auf eine möglichst einfache Ausgestaltung ist ein sich in Längsrichtung des Schlauchanschlusses in dem Körper erstreckende Abzugskanal vorgesehen, in den der Schlauchanschluss mündet. Der Schlauchanschluss und der Abzugskanal sind üblicherweise in Längsrichtung des Schlauchanschlusses hintereinander, besonders bevorzugt koaxial zueinander, vorgesehen.

Um ein Festsaugen des Zungenreinigers zu vermeiden, kommuniziert der Abzugskanal mit einer Lüftungsöffnung. Bei dieser Lüftungsöffnung handelt es sich um eine weitere Öffnung, die nicht der Schlauchanschluss ist. Vielmehr befindet sich die Lüftungsöffnung üblicherweise in Längsrichtung des Abzugskanals bzw. des Schlauchanschlusses an der dem Schlauchanschluss gegenüberliegenden Seite, an einem stirnseitigen Ende des Abzugskanals. Die Lüftungsöffnung ist so bemessen, dass bei dem üblicherweise wirkenden negativen Absaugdrücken der Zungenreiniger mit einer gewissen Haltekraft an der Zunge anliegend gehalten wird, in der er sich dort nicht festsaugen kann. So kann der Zungenreiniger zu jeder Zeit relativ zu der Zunge bewegt und von dieser abgezogen werden.

Die Unteransprüche beinhalten weitere Ausführungsbeispiele des erfindungsgemäßen Zungenreinigers.

Der Schlauchanschluss, der Abzugskanal und die Lüftungsöffnung sind in Achsrichtung des Zylinderstutzens vorzugsweise hintereinander angeordnet. Dementsprechend durchsetzt der von dem Abzugskanal und dem Zylinderstutzen gebildete Kanal üblicherweise den Körper mittig. Mit Blick auf eine möglichst flache, d.h. dünne Ausgestaltung des eigentlichen Körpers wird der Abzugskanal in einem konisch zulaufenden Rohrabschnitt ausgebildet, dessen durchmessergrößeres Ende an den Schlauchanschluss anschließt. Mit größerer Entfernung von dem Schlauchanschluss verringert sich dementsprechend der wirksame Strömungsquerschnitt des Abzugskanals. Aber selbst am Ende des Abzugskanals ist dessen Durchmesser üblicherweise erheblich größer als der wirksame Strömungsdurchmesser der Lüftungsöffnung. Diese befindet sich üblicherweise auf Höhe der äußeren Kontur des Körpers. Mit anderen Worten endet der konisch zulaufende Rohrabschluss endseitig zumindest im Wesentlichen bündig mit der Außenkontur der nutzungsseitigen Oberfläche. Vorzugsweise liegt die Lüftungsöffnung dabei innerhalb des umfänglich den Körper umgebenden Randes. Auf der gegenüberliegenden Seite ragt üblicherweise der Zylinderstutzen zur Ausbildung des Rohranschlusses ab. Jenseits dieses von dem Körper abragenden Zylinders und dem Rohrabschnitt ist der Körper scheibenförmig, üblicherweise elliptisch oder mit runder Grundform.

Gemäß einer bevorzugten weiteren Ausführungsform der Erfindung ist die Profilierung durch sich parallel zueinander erstreckende Lamellen gebildet. Dabei hat der Zungenreiniger beispielsweise zwischen vier und sechs Lamellen. Die Lamellen erstrecken sich üblicherweise rechtwinklig zu dem Abzugskanal und/oder dem Schlauchanschluss, d.h. dem Zylinderstutzen.

Die Lamellen gehen vorzugsweise von einer Lamellenbasis ab. Dabei sind die Lamellen dünner als die Lamellenbasis. Die Lamellenbasis ist dementsprechend gegenüber den Lamellen verbreitert. In einer Querschnittsansicht durch eine Lamelle ergibt sich dementsprechend ein im Wesentlichen L-förmiger Querschnitt, wobei die Basis des L durch die verbreiterte Lamellenbasis geformt wird und die eigentliche Lamelle als Steg von dieser absteht. Die Breite der Lamellenbasis kann bis zu 4 bis 8 Mal die Breite der Lamelle betragen. Als Breite wird hierbei die Erstreckung parallel zu der nutzungsseitigen Oberfläche bei einer Querschnittsansicht durch die Lamelle bzw. die Lamellenbasis verstanden. Bei der bevorzugten Ausgestaltung, bei welcher sich die Lamellen rechtwinklig zu dem Schlauchanschluss erstrecken, verläuft die besagte Schnittansicht in Längserstreckungsrichtung des Schlauchanschlusses, d.h. Zylinderstutzens.

Mit Blick auf eine effektive Reinigung der Zungenoberfläche sind mehrere Lamellen und/oder Lamellenbasen vorgesehen. Diese sind üblicherweise in Längsrichtung des Abzugskanals hintereinander angeordnet. Sie erstrecken sich üblicherweise jeweils parallel zueinander. Es ergibt sich dementsprechend eine Art Rost, wobei üblicherweise lediglich die freien Endflächen der Lamellen zur Anlage an die Oberfläche der Zunge gelangen, wohingegen die Lamellenbasen mit Abstand dazu und nach innen versetzt durch den Körper ausgebildet sind. Dabei ist die Ausgestaltung der Lamellenbasen üblicherweise die Elastizität bzw. Rückstellkraft der einzelnen Lamellen variiert. Jede Lamelle und die ihr zugeordnete Lamellenbasis sind üblicherweise einteilig ausgebildet und fest miteinander verbunden. Dabei kann jede Lamellenbasis in ihrer Erstreckungsrichtung einen sich ändernden Querschnitt bzw. eine sich ändernde Querschnittsform haben, um das Rückstellverhalten der ihr zugeordneten Lamelle zu beeinflussen. So kann die Stärke der Lamelle, d.h. üblicherweise ihre Erstreckung rechtwinklig zu der nutzungsseitigen Vorderfläche in Erstreckungsrichtung der Lamellenbasis, zu- oder abnehmen. Des Weiteren können Lamellen unterschiedlicher Position innerhalb der nutzungsseitigen Vorderfläche unterschiedlichen Funktionen zugewiesen sein. Hierzu wird gemäß einer bevorzugten weiteren Ausführungsform vorgeschlagen, die Stärke der Lamellenbasis in Erstreckungsrichtung des Schlauchanschlusses zur Mitte hin abnehmend auszubilden. Mit anderen Worten, hat die Lamellenbasis unmittelbar benachbart zu dem Schlauchanschluss bzw. am gegenüberliegenden Ende, d.h. in Erstreckungsrichtung des Schlauchanschlusses an dem Rand des Körpers, eine größere Stärke und sind danach biegesteifer als eine Lamellenbasis, die sich in etwa in der Mitte der nutzungsseitigen Vorderfläche in Erstreckungsrichtung des Schlauchanschlusses befindet, d.h. etwa den gleichen Abstand zu dem stirnseitigen Ende des Abzugskanals und dem gegenüberliegenden Ende desselben hat, dort, wo der Abzugskanal in den Schlauchanschluss mündet.

Vorzugsweise ist zumindest eine der Vorder- oder Rückwände der Lamelle konvex gekrümmt ausgebildet. Mit anderen Worten, ergibt sich in einer Schnittansicht in Erstreckungsrichtung des Schlauchanschlusses eine zumindest einseitige Balligkeit der Lamelle. Dabei verbreitern sich die Lamellen üblicherweise in Richtung auf ihren Lamellenfuß. Mit anderen Worten, haben die Lamellen an ihrem freien Ende eine geringere Breite als am Übergang der Lamelle zu der Lamellenbasis. Als Breite sei dabei diejenige Abmessung zu verstehen, die sich bei einer Schnittansicht in Erstreckungsrichtung des Schlauchanschlusses in jener Erstreckungsrichtung des Schlauchanschlusses erstreckt. Bei der konvex gekrümmten Vorder- oder Rückwand der Lamelle handelt es sich üblicherweise um diejenige Wand, die von der breiteren, der der jeweiligen Lamelle zugeordneten Lamellenbasis überragt ist. Die andere Seite endet üblicherweise bündig mit der Lamellenbasis und ist vorzugsweise geradlinig verlaufend ausgeformt.

Die Endflächen der Lamellen, d.h. die freie, üblicherweise die nutzungsseitige Vorderfläche des scheibenförmigen Körpers ausmachenden Endflächen der Lamellen sind vorzugsweise konvex gekrümmt. In ihrer Erstreckungsrichtung liegt die Lamelle dementsprechend mittleren Bereich, üblicherweise höher als an ihrem Rand, wo die Lamelle üblicherweise absatzfrei in den äußeren Rand des scheibenförmigen Körpers übergeht. So spannt jede Lamelle für sich eine konvexe Endfläche auf. Die Krümmung der mittleren Lamellen ist dabei üblicherweise stärker als die Krümmung der endseitigen Lamellen. Die Endflächen sämtlicher Lamellen liegen vorzugsweise in einer sphärisch ausgebildeten Hüllfläche. Die randseitigen Enden sämtlicher Lamellen enden üblicherweise flächenbündig mit dem die nutzungsseitige Vorderfläche umgebenden Rand. Der Rand definiert die unterste Ebene der nutzungsseitigen Oberfläche. Von diesem Rand ragen die Lamellen ab, die mit ihren gekrümmten Endflächen jeweils innerhalb der zuvor erwähnten sphärischen Hüllfläche liegen. Die sphärische Hüllfläche beinhaltet dabei üblicherweise die Oberfläche des umfänglich umlaufenden Randes.

Gemäß einer bevorzugten weiteren Ausführungsform der Erfindung ist zwischen einer Rückseite einer Lamelle und einer der hierzu benachbart vorgesehenen Lamelle zugeordneten Lamellenbasis wenigstens eine mit dem Abzugskanal kommunizierende Abzugsöffnung vorgesehen. Die Abzugsöffnung befindet sich dabei insbesondere in Verlängerung der Längserstreckung des Schlauchanschlusses. Üblicherweise liegen sämtliche Abzugsöffnungen auf einer Achse, die durch die Längserstreckung des Zylinderstutzens vorgegeben ist, d.h. sie liegen auf einer Mittellängsachse des Zylinderstutzens. Zu einer solchen Abzugsöffnung führt üblicherweise eine Ablaufrinne. Die Ablaufrinne ist zwischen einer Lamelle und einer Lamellenbasis vorgesehen, wobei die besagte Lamellenbasis einer anderen Lamelle zugeordnet ist, die benachbart zu der zuvor erwähnten Lamelle vorgesehen ist. Die Abzugsöffnung befindet sich dementsprechend üblicherweise zwischen einer Rückwand der Lamelle und einer Endfläche einer Lamellenbasis, die der jeweils benachbarten Lamelle zugeordnet ist. Es sind beidseitig jeder Abzugsöffnung jeweils Ablaufrinnen vorgesehen, die sich bis zu dem jeweiligen Rand erstrecken.

Die Ablaufrinne ist üblicherweise in Richtung auf die Abzugsöffnung geneigt ausgebildet. Mit anderen Worten, liegt ein seitlicher Rand der Ablaufrinne üblicherweise höher als die benachbart zu der Abzugsöffnung vorgesehene Endfläche der Ablaufrinne. Dabei liegt die Ablaufrinne üblicherweise nach innen versetzt zu der nutzungsseitigen Oberfläche der Lamellenbasis. Die Lamellenbasis geht verhältnismäßig scharfkantig, d.h. rechtwinklig in die Ablaufrinne über, wodurch unterhalb der durch die Endflächen der Lamellen gebildeten reinigungsaktiven Vorderfläche eine Profilierung zwischen der Lamellenbasis und der Ablaufrinne geschaffen ist, die gegebenenfalls noch etwas zur Reinigung der Zunge beitragen kann.

Schließlich wird gemäß einer bevorzugten weiteren Ausführungsform der vorliegenden Erfindung vorgeschlagen, in Erstreckungsrichtung des Schlauchanschlusses jeweils Abzugsöffnungen vor und hinter jeder der Lamellen vorzusehen. Dies führt zu einer sehr effektiven Abfuhr von durch die Profilierung gelöster Verschmutzung. Es ist üblicherweise eine Abzugsöffnung mehr als die Anzahl der Lamellen vorgesehen.

Weitere Einzelheiten der vorliegenden Erfindung ergeben sich aus der nachfolgenden Beschreibung eines Ausführungsbeispiels in Verbindung mit der Zeichnung.

In dieser zeigen:
- Fig. 1: eine perspektivische Seitenansicht des Ausführungsbeispiels;
- Fig. 2: eine weitere perspektivische Draufsicht auf das Ausführungsbeispiel
- Fig. 3: eine perspektivische Ansicht entsprechend Fig. 1 bei Schnitt durch die Mittellängsachse;
- Fig. 4: eine Schnittdarstellung entlang der Linie IV-IV gemäß der Darstellung in Fig. 2;
- Fig. 5: eine Schnittdarstellung gemäß der Linie V-V gemäß der Darstellung in Fig. 2;
- Fig. 6: eine geschnittene Seitenansicht entlang der Linie VI-VI gemäß der Darstellung in Fig. 4 für ein anderes Ausführungsbeispiel mit geradlinig verlaufenden Rückwänden der Lamellen,
- Fig. 7: eine geschnittene Seitenansicht entlang der Linie VII-VII gemäß der Darstellung in Fig. 4 für ein anderes Ausführungsbeispiel mit geradlinig verlaufenden Rückwänden der Lamellen;
- Fig. 8: eine Draufsicht eines zweiten Ausführungsbeispiels der vorliegenden Erfindung;
- Fig. 9: eine perspektivische unterseitige Ansicht des zweiten Ausführungsbeispiels;
- Fig. 10: eine Seitenansicht des zweiten Ausführungsbeispiels;
- Fig. 11: eine stirnseitige Ansicht des zweiten Ausführungsbeispiels;
- Fig. 12: .eine Schnittdarstellung entlang der Linie XII-XII gemäß der Darstellung in Fig. 9;
- Fig. 13: eine Querschnittsansicht entlang der Linie XIII-XIII gemäß der Darstellung in Fig. 9 und
- Fig. 14: eine vergrößerte Schnittdarstellung eines Randbereiches des zweiten Ausführungsbeispiels

In dieser Zeichnung kennzeichnet Bezugszeichen 2 einen scheibenförmigen Körper 2, der einseitig von einem Zylinderstutzen 4 überragt ist, welcher einen Schlauchanschluss 6 ausformt. Der Zylinderstutzen 4 setzt sich in seiner Längserstreckung fort in einem Abzugskanal 8, der in einem konisch zulaufenden Rohrabschnitt 10 ausgeformt ist. Der Rohrabschnitt 10 und der Zylinderstutzen 4 haben an ihrer Außenseite eine glatte Kontur, d.h. gehen absatzfrei ineinander über. Der Rohrabschnitt 10 endet an seinem dem Zylinderstutzen 4 gegenüberliegenden Ende in einer stirnseitigen Abschlusswandung 12, die mit einer Lüftungsöffnung 14 versehen ist. Bei den gezeigten Ausführungsbeispiel sind der Schlauchanschluss 6, der Abzugskanal 8 und die Lüftungsöffnung 14 koaxial zueinander. Zwischen dem Schlauchanschluss 6 und dem Abzugskanal 8 ist eine Ringfläche 16 ausgeformt, die der endseitigen Anlage und Abdichtung eines in den Schlauchanschluss 6 eingebrachten Schlauches dient.

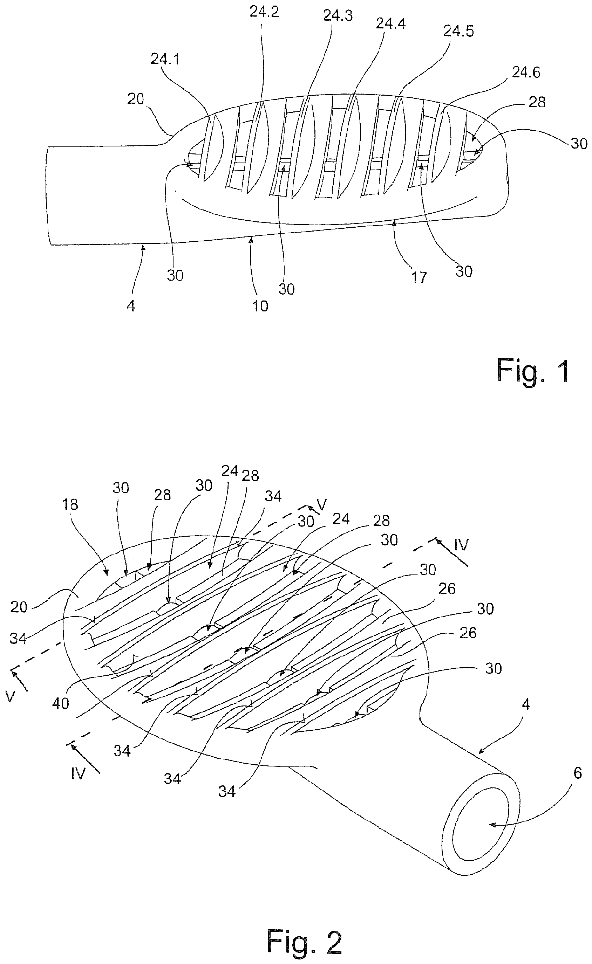

Wie insbesondere aus Fig. 2 ersichtlich, ist der scheibenförmige Körper 2 tellerförmig ausgebildet und lediglich auf einer Rückseite 17 von dem Zylinderstutzen 4 und dem konisch zulaufenden Rohrabschnitt 10 überragt. Eine dazu gegenüberliegende nutzungsseitige Vorderfläche 18 des scheibenförmigen Körpers ist hingegen im Wesentlichen eben, wozu auch beiträgt, dass der Zylinderstutzen 4 an dieser Vorderfläche 18 flächenbündig mit einem umfänglich umlaufenden Rand 20 des scheibenförmigen Körpers 2 ausgebildet ist. Dieser umlaufende Rand 20 hat - in Querschnittsansicht des Randes 20 - eine gleichmäßige konvexe Krümmung, um eine Verletzung der empfindlichen Mundschleimhäute auszuschließen.

Innerhalb dieses Randes 20 befindet sich eine Profilierung 22, bestehend aus Lamellen 24, die sich von einer Lamellenbasis 26 erheben. Zwischen einer der Lamellenbasen 26 und einer Lamelle 24 mit einer anderen Lamellenbasis 26 befindet sich ein Ablaufrinne 28, die zu einer Abzugsöffnung 30 führt. Sämtliche Abzugsöffnungen 30 sind in Verlängerung der Längserstreckung des Zylinderstutzens 4/Schlauchanschlusses 6 vorgesehen. Die Ablaufrinne 28 wird jeweils durch einen relativ dünnwandigen Steg 32 gebildet, der zwischen einer Lamellenbasis 26 und einer benachbart hierzu vorgesehen anderen Lamelle 24 angeordnet ist.

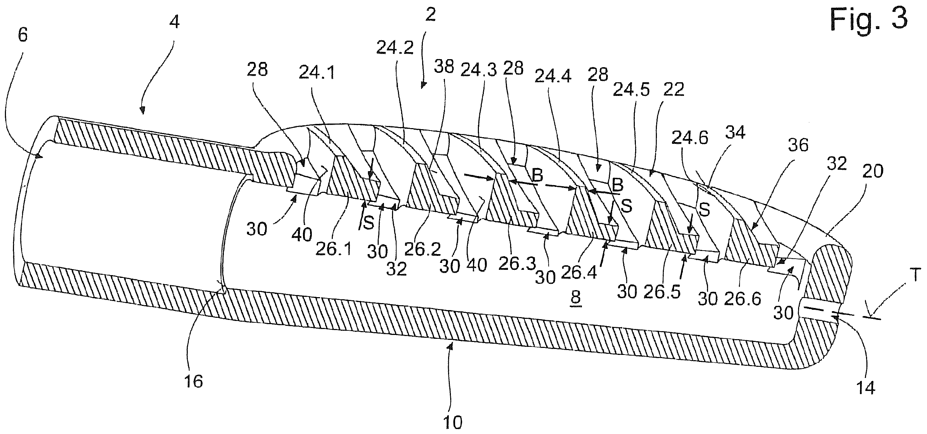

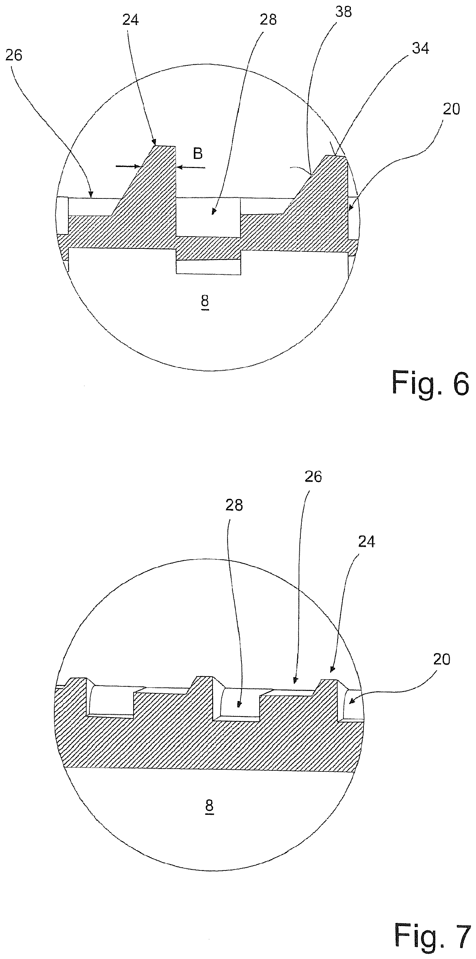

Wie ersichtlich, erstrecken sich die Lamellen 26 rechtwinklig zu der Erstreckungsrichtung des Zylinderstutzens 4 des Schlauchanschlusses 6. Die Lamellen 24 und die ihnen jeweils zugeordneten Lamellenbasen 26 sind im Querschnitt L-förmig ausgeformt. So verlaufen die Lamellen und die ihr zugeordnete Lamellenbasis streng parallel zueinander. Wie die Schnittansichten gemäß Fig. 3 und 6 verdeutlichen, ist die Lamellenbasis 26 gegenüber der ihr zugeordneten Lamelle 24 verbreitert. Die Breite der Lamelle 24 ist in Fig. 3B eingezeichnet. Die Lamelle 24 hat eine geringere Breite als die Lamellenbasis 26. Dadurch ergibt sich im Grunde eine L-förmige Querschnittsgestaltung von Lamelle 24 und dieser zugeordneten Lamellenbasis 26.

Jede einzelne Lamelle 24 hat eine konvex gekrümmte Endfläche 34. Mit anderen Worten, geht die Endfläche 34 jeder einzelnen Lamelle 24 von dem umlaufenden Rand 20 ab und ist höhenbündig mit diesem ausgebildet. Die Lamelle 24 steigt aber zur Mitte des scheibenförmigen Körpers 2 hin an. Mit anderen Worten liegt die Endfläche 34 auf Höhe des Abzugskanals 8 höher als am Rand 20. Darüber hinaus haben die mittleren Lamellen 24 eine höhere Höhe als die anderen Lamellen 24. Hiermit ergibt sich eine die Endflächen 34 enthaltende Hüllfläche, die sphärisch ausgestaltet ist und die die nutzungsseitige Oberfläche des Randes 20 beinhaltet. Wie weiterhin aus Fig. 6 ersichtlich, verbreitert sich jede Lamelle 24 zu ihrem Lamellenfuß 36 hin. Die Lamelle 24 ist danach schmaler im Bereich ihrer Endfläche 34 relativ zu derjenigen Stelle, wo die Lamelle 24 in die Lamellenbasis 26 übergeht. Diese Verbreiterung wird vorwiegend durch eine konvex gekrümmte Rückwand 38 der Lamellen 24 ausgebildet. Die konvexe Krümmung an der Rückwand 38 variiert indes in Erstreckungsrichtung der Lamelle 24. So ist die Balligkeit der Lamelle nahe dem Rand 20 geringer als diejenige in der Mitte, d.h. auf Höhe des Schlauchanschlusses 6 bzw. in axialer Verlängerung davon und auf Höhe des Abzugskanals 8. So nimmt die Breite B der Lamelle 24 zu ihrer Mitte hin kontinuierlich zu. Dementsprechend hat auch die Lamelle 24 ihre breiteste Stelle in axialer Verlängerung des Schlauchanschlusses.

Während die Rückwand 38 der Lamelle 24 konvex gekrümmt ist, verläuft eine Vorderwand 40 der Lamelle 24 geradlinig, wodurch sich ein L-förmiger Querschnitt für die Einheit aus Lamelle 24 und zugeordneter Lamellenbasis 26 ergibt.

Wie insbesondere der Schnittdarstellung gemäß Fig. 3 zu entnehmen ist, variiert die Stärke S der Lamellenbasis 26 in Erstreckungsrichtung des Schlauchanschlusses 6. Die Stärke S nimmt dabei insbesondere in Erstreckungsrichtung des Schlauchanschlusses 6 zur Mitte des Körpers 2 hin ab. Randseitige Lamellenbasen 26.1 und 26.6 haben dementsprechend eine stärkere Stärke S als die mittleren Lamellenbasen 26.3 und 26.4.

Die Lamellen 24 und die Lamellenbasen 26 sind endseitig flächenbündig mit dem Rand 20 vorgesehen. Dementsprechend ergibt sich am Rand 20 eine absatzfreie und glatte Kontur. Die zwischen einer Lamelle 24.2 und einer der benachbart hierzu vorgesehenen Lamelle 24.1 zugeordneten Lamellenbasis 26.1 vorgesehene Ablaufrinne 28 endet unterhalb der nutzungsseitigen Oberfläche des Randes 20.

Jede Ablaufrinne 28 führt zu der zugeordneten Abzugsöffnung 30 und ist durch diese mittig geteilt. Die Ablaufrinne 28 ist geneigt ausgebildet, d.h. neigt sich nach unten in Richtung auf die zugeordnete Abzugsöffnung 30.

Wie die Figuren verdeutlichen, hatte das gezeigte Ausführungsbeispiel sechs Lamellen 24 mit ihren zugeordneten Lamellenbasen 26. Es sind indes sieben Abzugsöffnungen 30 vorgesehen. Jede der Einheit aus Lamelle 24 und Lamellenbasis 26 ist vorderseitig und rückseitig zwischen zwei Abzugsöffnungen 30 vorgesehen.

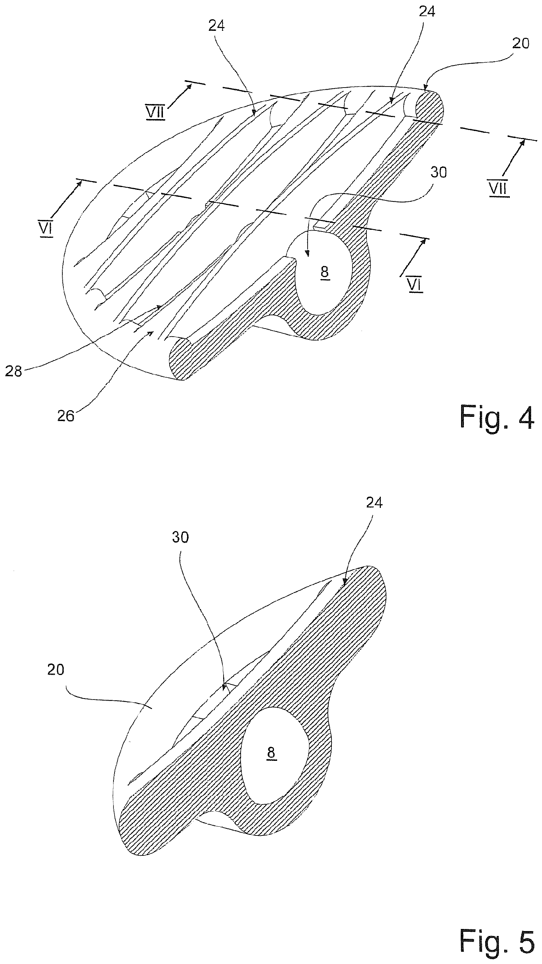

Wie insbesondere Fig. 4 verdeutlicht, ist der scheibenförmige Körper 2 relativ dünn, d.h. tellerförmig ausgebildet und lediglich rückseitig von dem Zylinderstutzen 4 bzw. dem konisch zulaufenden Rohrabschnitt 10 überragt. So lässt sich das gezeigte Ausführungsbeispiel gut in den Mundraum des menschlichen Körpers einbringen. Die Breite, d.h. Erstreckungsrichtung des scheibenförmigen Körpers 2 in Längsrichtung des Schlauchanschlusses beträgt vorzugsweise zwischen 26 und 33 cm (ohne die nutzungsseitige Vorderfläche 18 überragenden Zylinderstutzen 4. In einer Richtung rechtwinklig hierzu hat die Vorderfläche 18 eine Erstreckung von zwischen 23 und 28 cm.

Das gezeigte Ausführungsbeispiel ist als Spritzgussteil hergestellt. Dabei wird der Schlauchanschluss 6 und der Abzugskanal 8 durch einen beweglichen Kern freigelassen, der in eine Spritzgussform eingebracht ist, deren Teilungsebene sich im Wesentlichen parallel zu der nutzungsseitigen Vorderfläche 18 erstreckt und sich auf Höhe der größten Breite des Zylinderstutzens 4 befindet. Die Teilungsebene ist in Fig. 3 mit T gekennzeichnet. Die Lamellen 24 erstrecken sich rechtwinklig zu der Teilungsebene T, so dass das in den Figuren verdeutlichte Ausführungsbeispiel eines einheitlichen Spritzgusskörpers nach dem Öffnen der Werkzeughälften ohne weitere Hilfsmittel entformt werden kann.

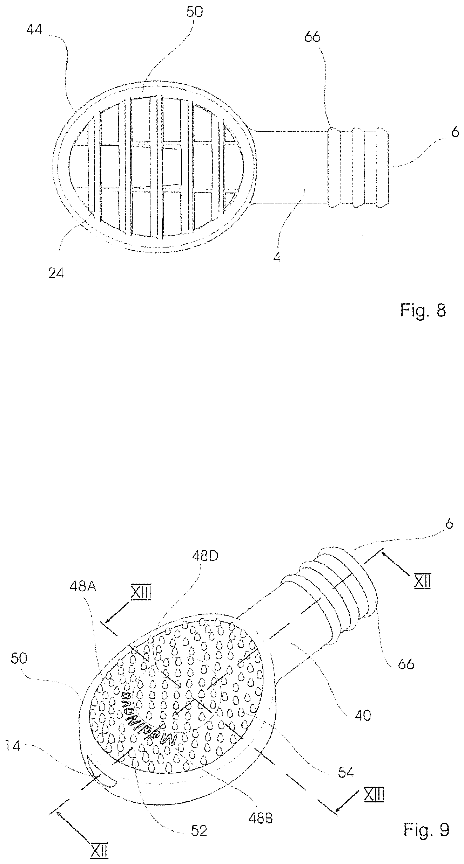

Das in den Figuren 8 bis 14 gezeigte zweite Ausführungsbeispiel ist ein Zungenreiniger, der mittels Zweikomponenten-Spritzgießen hergestellt ist und eine mit Bezugszeichen 42 gekennzeichnete Weichkomponente umfasst, die durch Umspritzen eines Basiskörpers aus einer mit Bezugszeichen 44 gekennzeichneten Hartkomponente ausgeformt ist. Die Weichkomponente hat dabei vorzugsweise eine Härte Shore A von zwischen 25 und 80 und ist vorzugsweise aus TPE gebildet. Die Hartkomponente ist vorzugsweise PP und hat üblicherweise eine Härte Shore A von zwischen 70 und 100. Entsprechend dem Bezugszeichen 44 für die Hartkomponente wird auch der durch die Hartkomponente gebildete Grundkörper mit diesem Bezugszeichen gekennzeichnet, wohingegen Bezugszeichen 42 die Weichkomponente und damit auch die Umspritzung verdeutlicht. Ansonsten wurden die in der vorherigen Beschreibung unter Bezugnahme auf das erste Ausführungsbeispiel verwendeten Bezugszeichen - wo möglich - beibehalten.

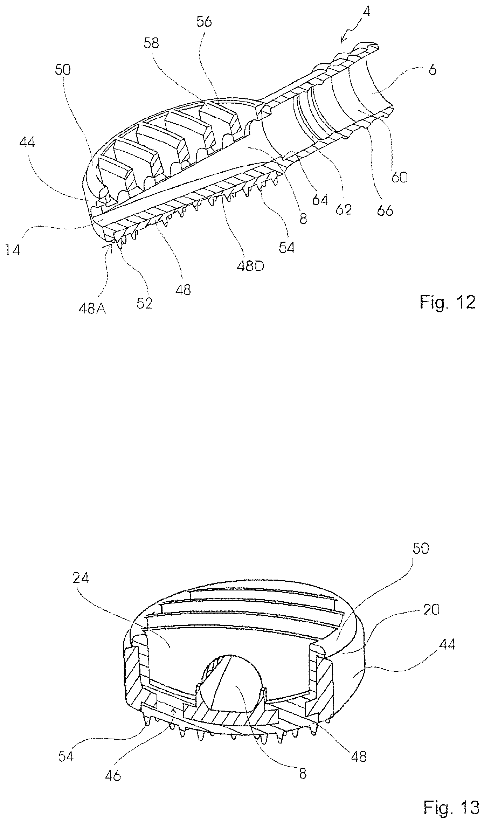

Wie insbesondere die Figuren 12 und 13 verdeutlichen, ist der Grundkörper 44 im Bereich des scheibenförmigen Körpers 2 schalenförmig ausgebildet und hat vorliegend mehrere, üblicherweise zumindest eine Durchspritzöffnung 46. Über diese Durchspritzöffnung 46 ist ein Kissen 48 an der Rückseite 17 einteilig mit einem elastomeren Schutzrand 50 verbunden, der den Rand 20 des Grundkörpers 44 überragt und im Wesentlichen umgibt. Auch die Lamellen 24 sind aus der Weichkomponente gebildet. Vorliegend sind die Lamellen 24 ausschließlich aus der Weichkomponente 42 gebildet. Es sind aber auch Ausgestaltungen denkbar, bei welchen ein Gerippe aus der Hartkomponente 49 von einer Weichkomponente 42 überzogen wird, um die Rippen 24 auszubilden.

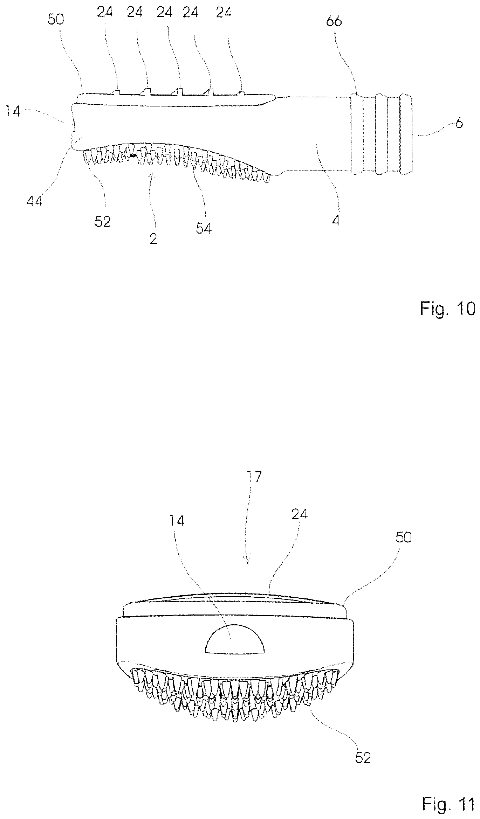

Wie insbesondere Figur 11 verdeutlicht, weist das Kissen 48 eine im Grunde ebene, leicht nach außen konvex gekrümmte Gestaltung auf. Entgegen dem ersten Ausführungsbeispiel fehlt es bei dieser zweiten Ausgestaltung an einem rückseitig vorspringenden Rohrabschnitt. Vielmehr ist der Abzugskanal 8 innerhalb des scheibenförmigen Körpers 2 aufgenommen. Von dem Kissen 48 ragen Noppen 52, 54 unterschiedlicher Länge ab. Bei den Massagenoppen mit dem Bezugszeichen 52 handelt es sich um die langen, bei den Massagenoppen mit dem Bezugszeichen 54 handelt es sich um die kurzen Noppen. Die langen Noppen 52 sind vorliegend etwa doppelt so lang wie die kurzen Noppen 54. Üblicherweise können die Noppen 52, 54 eine Bauhöhe von zwischen 0,5 und 1,5 mm haben. Die Noppen 52, 54 können verschiedene Ausgestaltungen aufweisen. Zu bevorzugen sind Noppen mit kreisrundem Querschnitt. Deren Durchmesser liegt vorzugsweise zwischen 0,3 und 2,0 mm. Das rückseitige Kissen 48 mit den Noppen 52, 54 dient der Massage des Zungengrundes. Mit einem solchen Kissen 48 können Verschmutzungen im Mundraum gelöst werden, die durch den Zungenreiniger danach abgesaugt werden können. Auch ist es möglich, bei einer einmaligen Bewegung durch den Mundraum unterschiedliche Flächen zu reinigen, nämlich solche, an denen das Kissen 48 vorbeigeführt wird, und solche, an denen die Lamellen 24 vorbeigeführt werden.

Des Weiteren kann das Kissen 48 mit den Noppen 52, 54 als Applikator zum Aufbringen von Reinigungs- oder Desinfektionsmitteln auf die Zunge verwendet werden. Das Kissen 48 weist hierzu ein Noppenfeld mit zwischen 30 und 80 Noppen 52, 54 auf.

Hierzu weist das Kissen 48 einen mittleren Dosierbereich 48D mit verhältnismäßig geringer Wandstärke auf Seiten des Kissens 48 und relativ langen Noppen 52, 54 und einen diesen umfänglich umgebenden Applikationsbereich 48A auf. Der Dosierbereich 48D dient beispielsweise dem Aufbringen einer einzumassierenden Pflegesubstanz. Wie des Weiteren Figur 9 verdeutlicht, kann am Rand zwischen dem Dosierbereich 48D, der vorliegend kreisförmig ausgebildet ist, und bereits in dem Applikationsbereich 48A ein Branding 48B, d.h. eine Marke oder dergleichen vorgesehen sein.

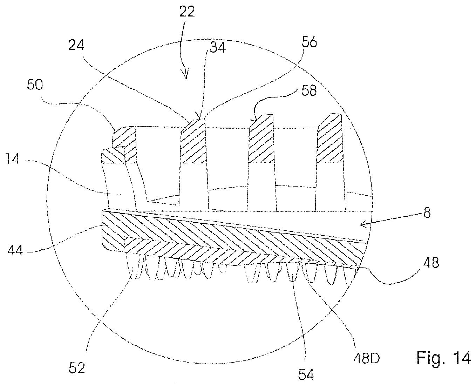

Es können zwischen eins und zehn Lamellen vorgesehen sein. Bei dem zweiten Ausführungsbeispiel sind fünf Lamellen 24 verwirklicht. Wie Figur 14 verdeutlicht, wird durch die Profilierung 22 der Lamellen 24 eine Scharbkante 56 ausgebildet, die auf der dem Schlauchanschluss 4 zugewandten Flanke der Lamelle zwischen dieser Flanke und der Endfläche 34 ausgeformt wird, sowie eine Schrägfläche 58, die zwischen der Endfläche 34 und der der Lüfungsöffnung 14 zugewandten Flanke an der Lamelle 24 vorgesehen ist. Diese Ausgestaltung hat sich als vorteilhaft für eine gründliche Reinigung beim Scharben der Zunge erwiesen. Dabei kann die Scharbkante (56) hartnäckigere Verschmutzung von der Zunge abheben. Die Schrägfläche 58 verbessert das Abführen der so losgelösten Verschmutzung in den Abzugskanal 8.

Wie die Figuren 9 und 11 verdeutlichen, hat die Lüftungsöffnung 14 einen sichelförmigen bzw. halbkreisförmigen Querschnitt und ist - wie das Profil des Abzugskanals 8 (vgl. Fig. 13) - in Richtung auf die freien Enden der Lamellen 24 vorgewölbt ausgebildet. Diese Ausgestaltung verbessert das Absaugen von gelöster Verschmutzung und verhindert u.a., dass abgesaugtes Sekret wieder zurückströmen kann. Die Lüftungsöffnung 14 ermöglicht einen Betrieb des Ausführungsbeispiels ohne übermäßige Beanspruchung der Gewebeoberfläche mit einem nicht regulierbaren Absaugdruck, wie er üblicherweise bei zahnärztlichen Arbeitsplätzen zur Verfügung steht. Dabei besteht ein proportionales Verhältnis zwischen der Absaugfläche, d.h. der innerhalb des Randes 20 eingeschlossenen Freifläche und dem Querschnitt der Lüftungsöffnung 14. Die Absaugfläche kann dabei zwischen 5 und 50 mal größer sein als der Querschnitt der Lüftungsöffnung 14. Auch bei dem zweiten Ausführungsbeispiel befindet sich die Lüftungsöffnung 14 in Verlängerung des Abzugskanals 8, um in jedem Fall einen gewissen Saugdruck an der Absaugfläche bereitzustellen, d.h. die Absaugfläche zwischen der Lüftungsöffnung 14 und dem Schlauchanschluss 6 zu platzieren. Hierdurch wird auch der Effekt erzielt, dass der Absaug-Luftstrom über die Gewebefläche geleitet wird und dort eine funktionale Wirkung zeigt. Gleichwohl könnte die Lüftungsöffnung 14 an einer anderen geeigneten, nicht erfindungsgemäßen Stelle vorgesehen sein. Bei dem gezeigten Ausführungsbeispiel wird durch die Ausgestaltung und Anordnung der Lüftungsöffnung 14 gewährleistet, dass eine gleichmäßige, in allen Bereichen der Absaugfläche gleich starke Absaugleistung bereitgestellt wird.

Die Figur 12 verdeutlicht die Details des Schlauchanschlusses 6, der mit geringem axialem Abstand von der Mündungsöffnung eine Einführschräge 60 aufweist, um den Schlauch, bei dem es sich üblicherweise um einen standardmäßig in der Zahnmedizin verwendeten Schlauch mit einem Durchmesser von ca. 6 mm handelt, eng zu führen. Mit weiterem axialen Abstand von der Einführöffnung befinden sich mehrere Halterippen 62, die den dort eingeführten Schlauch umfänglich klemmen und halten. Des Weiteren ist am Ende des Schlauchanschlusses 6 ein Anschlag 64 vorgesehen, an dem die Einführbewegung des Schlauches ihr Ende findet. Der Anschlag 64 markiert den Beginn des Abzugskanals 8. Dieser Abzugskanal 8 mündet üblicherweise absatzfrei in den an dem Anschlag 64 anliegenden Schlauch, d.h. das Lumen des Schlauches. Der Zylinderstutzen 4 hat ferner an seiner Außenumfangsfläche mehrere axial beabstandete Griffrippen 66, mit welchen der Zungenreiniger zum Aufschieben auf den Schlauch besser und sicherer gehalten werden kann.

Vorzugsweise sind zwischen einer und drei solcher Griffrippen 66 vorgesehen.

Wie die Figuren 12 und 14 verdeutlichen, sind die Lamellen 24 im Grunde mit identischer Stärke ausgebildet und münden unmittelbar in den Abzugskanal 8. Die unter Bezugnahme auf das erste Ausführungsbeispiel näher erläuterte Profilierung der Lamellen 24 fehlt. Vielmehr ragen die Lamellen 24 als "Rippen" über dem Abzugskanal 8 mit leicht konvex gekrümmtem Verlauf quer zu der Längserstreckung des Abzugskanals 8 über den umlaufenden Schutzrand 50 hervor, gehen indes absatzfrei in diesen Schutzrand 50 über.

### Bezugszeichenliste

- 2: Scheibenförmiger Körper
- 4: Zylinderstutzen
- 6: Schlauchanschluss
- 8: Abzugskanal
- 10: Rohrabschnitt
- 12: Abschlusswandung
- 14: Lüftungsöffnung
- 16: Ringfläche
- 17: Rückseite
- 18: Vorderfläche
- 20: Rand
- 22: Profilierung
- 24: Lamelle
- 26: Lamellenbasis
- 28: Ablaufrinne
- 30: Abzugsöffnung
- 32: Steg
- 34: Endfläche
- 36: Lamellenfuß
- 38: Rückwand
- 40: Vorderwand
- 42: Weichkomponente/Umspritzung
- 44: Hartkomponente/Grundkörper
- 46: Durchspritzöffnung
- 48: Kissen
- 48A: Applikationsbereich
- 48B: Brandingbereich
- 48D: Dosierbereich
- 50: Schutzrand
- 52: Massagenoppen, lang
- 54: Massagenoppen, kurz
- 56: Scharbkante
- 58: Schrägfläche
- 60: Einführschräge
- 62: Halterippe
- 64: Anschlag
- 66: Griffrippe
- B: Breite der Lamelle
- S: Stärke der Lamellenbasis
- T: Teilungsebene

## Patentansprüche

1. Zungenreiniger mit einem im Wesentlichen scheibenförmigen Körper (2), der auf seiner nutzungsseitigen Vorderfläche (18) mit einer zum Abschaben von oberflächlich an der menschlichen Zunge anhaftender Verschmutzung geeigneten Profilierung (22) versehen ist und einen Schlauchanschluss (6) ausformt, der mit der Profilierung (22) kommuniziert, **dadurch gekennzeichnet,**
**dass** der Schlauchanschluss (6) in einen sich in Längsrichtung des Schlauchanschlusses (6) in dem Körper (2) erstreckenden Abzugskanal (8) mündet, der mit einer Lüftungsöffnung (14) kommuniziert, die in Längsrichtung des Abzugskanals an einer dem Schlauchanschluss (6) gegenüberliegenden stirnseitigen Ende des Abzugskanals vorgesehen ist.

2. Zungenreiniger nach Anspruch 1, **dadurch gekennzeichnet, dass** der Schlauchanschluss (6) durch einen eine Rückseite (17) des Körpers (2) überragenden Zylinderstutzen (4) ausgeformt ist.

3. Zungenreiniger nach einem der vorherigen Ansprüche, **dadurch gekennzeichnet, dass** der Schlauchanschluss (6), der Abzugskanal (8) und die Lüftungsöffnung (14) in Achsrichtung des Zylinderstutzens (4) hintereinander angeordnet sind.

4. Zungenreiniger nach einem der vorherigen Ansprüche, **dadurch gekennzeichnet, dass** der Abzugskanal (8) in einem konisch zulaufenden Rohrabschnitt (10) ausgebildet ist, dessen durchmessergrößeres Ende an den Schlauchanschluss (6) anschließt.

5. Zungenreiniger nach einem der vorherigen Ansprüche, **dadurch gekennzeichnet, dass** die Profilierung (22) sich parallel zueinander erstreckende Lamellen (24) aufweist die sich rechtwinklig zu dem Abzugskanal (8) und/oder dem Schlauchanschluss (6) erstrecken.

6. Zungenreiniger nach Anspruch 5, **dadurch gekennzeichnet, dass** die Lamellen (24) von einer Lamellenbasis (26) abgehen, die gegenüber der ihr zugeordneten Lamellen (24) verbreitert ist.

7. Zungenreiniger nach einem der Ansprüche 5 bis 6, **dadurch gekennzeichnet, dass** die freien Enden der Lamellen (24) eine konvex gekrümmte Endfläche (34) aufweisen und dass die Endflächen (34) sämtlicher Lamellen (24) in einer sphärisch ausgebildeten Hüllfläche liegen.

8. Zungenreiniger nach einem der Ansprüche 5 bis 7, **dadurch gekennzeichnet, dass** zumindest eine der Vorder- oder Rückwände (40; 38) der Lamellen (24) geradlinig verlaufend ausgebildet ist.

9. Zungenreiniger nach einem der Ansprüche 5 bis 8, **dadurch gekennzeichnet, dass** die Stärke (S) der Lamellenbasis (26) in Erstreckungsrichtung des Schlauchanschlusses (6) zur Mitte hin zunimmt.

10. Zungenreiniger nach einem der Ansprüche 5 bis 9, **dadurch gekennzeichnet, dass** zwischen einer Rückseite einer Lamelle (24) und einer der hierzu benachbart vorgesehenen Lamelle (24) zugeordneten Lamellenbasis (26) wenigstens eine mit dem Abzugskanal (8) kommunizierende Abzugsöffnung (30) vorgesehen ist und dass zwischen einer der Lamellen (24) und einer der hierzu benachbart vorgesehenen Lamelle (24) zugeordneten Lamellenbasis (26) eine zu der Abzugsöffnung (30) führende Ablaufrinne (28) vorgesehen ist.

11. Zungenreiniger nach Anspruch 10, **dadurch gekennzeichnet, dass** die Ablaufrinne (28) in Richtung auf die Abzugsöffnung (30) geneigt ausgebildet ist und dass die Ablaufrinne (28) randseitig unterhalb eines die nutzungsseitige Vorderfläche (18) umgebenden Randes (20) endet, der die nutzungsseitige Vorderfläche (18) umgibt und flächenbündig zu einer Lamelle (24) und/oder einer Lamellenbasis (26) vorgesehen ist.

12. Zungenreiniger nach einem der Ansprüche 5 bis 11, **dadurch gekennzeichnet, dass** in Erstreckungsrichtung des Schlauchanschlusses (6) Abzugsöffnungen (30) jeweils vor und hinter den Lamellen (24) vorgesehen sind.

13. Zungenreiniger nach einem der vorherigen Ansprüche, **dadurch gekennzeichnet, dass** der Körper (2) im Wesentlichen tellerförmig ausgebildet und an der Rückseite (17) lediglich von dem Zylinderstutzen (4) und dem konisch zulaufenden Rohrabschnitt (10) überragt ist.

14. Zungenreiniger nach einem der vorherigen Ansprüche, **gekennzeichnet durch** einen Grundkörper (44), der mit einer zumindest die Profilierung ausbildenden Umspritzung aus einer Weichkomponente versehen ist.

15. Zungenreiniger nach einem der vorherigen Ansprüche, **dadurch gekennzeichnet, dass** die Umspritzung die Profilierung wie auch ein an der Rückseite des Zungenreinigers ausgebildetes Kissen (48) ausformt und dass das Kissen (48) aus einer anderen Kunststoffkomponente, insbesondere einer anderen Weichkomponente als die Profilierung (22) gebildet ist.

16. Zungenreiniger nach einem der vorherigen Ansprüche, **dadurch gekennzeichnet, dass** an der Rückseite (17) des scheibenförmigen Körpers (2) ein mit Noppen (52, 54) versehendes Kissen (48) vorgesehen ist und dass das Kissen (48) aus einer Weichkomponente gebildet ist.

## Claims

1. Tongue cleanser having an essentially disc-shaped body (2), which on its use-side front surface (18) is provided with a profiling (22) suitable for superficially scraping impurity adherent at the human tongue and forming a hose connection (6), which communicates with the profiling (22),
**characterized in that**
the hose connection (6) merges in longitudinal direction of the hose connection (6) inside the body (2) an extending outlet duct (8) which communicates with a ventilation opening (14) that is arranged in longitudinal direction of the outlet duct on the front end of the outlet duct opposite to the hose connection (6).

2. Tongue cleanser according to claim 1, **characterized in that** the hose connection (6) is formed by a cylinder nozzle (4) surpassing a rear side (17) of the body (2).

3. Tongue cleanser according to one of the preceding claims, **characterized in that** the hose connection (6), the outlet duct (8), and the ventilation opening (14) are arranged in axial direction of the cylinder nozzle (4) one after another.

4. Tongue cleanser according to one of the preceding claims, **characterized in that** the outlet duct (8) is formed in a conically tapered tube section (10), the end with the greater diameter of which connects the hose connection (6).

5. Tongue cleanser according to one of the preceding claims, **characterized in that** the profiling (22) has fins (24) extending parallel to one another that extend perpendicularly to the outlet duct (8) and/or the hose connection (6).

6. Tongue cleanser according to claim 5, **characterized in that** the fins (24) route from a fin base (26), which is widened compared to the fins (24) associated to the same.

7. Tongue cleanser according to one of the claims 5 to 6, **characterized in that** the free ends of the fins (24) have a convex bent end surface (34) and that the end surfaces (34) of all fins (24) are lying inside a spherically shaped shell surface.

8. Tongue cleanser according to one of the claims 5 to 7, **characterized in that** at least one of the front or rear walls (40; 38) of the fins (24) are linearly running configured.

9. Tongue cleanser according to one of the claims 5 to 8, **characterized in that** the strength (S) of the fin base (26) increases towards the center in extension direction of the hose connection (6).

10. Tongue cleanser according to one of the claims 5 to 9, **characterized in that** between a rear side of a fin (24) and a fin base (26) associated to the fin (24) provided adjacently thereto, there is provided at least one outlet duct (8) communicating with the vent opening (30) and that between a fin (24) and a fin base (26) associated to the fin (24) provided adjacently thereto, there is provided a drain channel (28) leading to the vent opening (30).

11. Tongue cleanser according to claim 10, **characterized in that** the drain channel (28) is formed inclined in direction of the vent opening (30) and that the drain channel (28) ends at the lateral edge below the edge (20) encompassing the use-side front surface (18) and the fin (24) and/or the fin base (26) at the end are arranged flush with the edge (20).

12. Tongue cleanser according to one of the claims 5 to 11, **characterized in that** in extension direction of the hose connection (6), vent openings (30) are provided before and behind the fins (24), respectively.

13. Tongue cleanser according to one of the preceding claims, **characterized in that** the body (2) is basically plate-shaped configured and at the rear side (17) is only surpassed by the cylinder nozzle (4) and the conically tapered tube section (10).

14. Tongue cleanser according to one of the preceding claims, **characterized by** a base body (44), which is provided with an insert-molding made of a soft-elastic component at least forming the profiling.

15. Tongue cleanser according to one of the preceding claims, **characterized in that** the insert-molding forms the profiling as well as a pad (48) arranged at the rear side of the tongue cleanser and that the pad (48) is formed of a different plastic component, in particular of a different soft component than the profiling (22).

16. Tongue cleanser according to one of the preceding claims, **characterized in that** at the rear side (17) of the disc-shaped body (2), there is provided a pad (48) comprising nubs (52, 54) and that the pad (48) is made of a soft component.

## Revendications

1. Gratte-langue ayant un corps (2) sensiblement en forme de disque, qui est pourvu côté utilisation sur sa surface avant (18) d'un profilé (22) approprié pour racler les salissures adhérant superficiellement à la langue humaine et qui forme un raccord de tube (6) qui communique avec le profilé (22), **caractérisé en ce que** le raccord de tube (6) débouche dans un canal d'évacuation (8) s'étendant dans le corps (2) dans le sens longitudinal du raccord de tube (6), qui communique avec une ouverture d'aération (14) qui est prévue dans le sens longitudinal du canal d'évacuation à une extrémité avant du canal d'évacuation opposée au raccord de tube (6).

2. Gratte-langue selon la revendication 1, **caractérisé en ce que** le raccord de tube (6) est formé d'un embout cylindrique (4) qui dépasse d'une face arrière (17) du corps (2).

3. Gratte-langue selon l'une des revendications précédentes, **caractérisé en ce que** le raccord de tube (6), le canal d'évacuation (8) et l'ouverture d'aération (14) sont disposés l'un derrière l'autre dans le sens axial de l'embout cylindrique (4).

4. Gratte-langue selon l'une des revendications précédentes, **caractérisé en ce que** le canal d'évacuation (8) est formé dans une section de tube conique (10), dont l'extrémité de plus grand diamètre est raccordée au raccord de tube (6).

5. Gratte-langue selon l'une des revendications précédentes, **caractérisé en ce que** le profilé (22) présente des lamelles (24) s'étendant parallèlement les unes aux autres, qui s'étendent perpendiculairement au canal d'évacuation (8) et/ou au raccord de tube (6).

6. Gratte-langue selon la revendication 5, **caractérisé en ce que** les lamelles (24) partent d'une base de lamelles (26) qui est élargie par rapport aux lamelles (24) qui lui sont associées.

7. Gratte-langue selon l'une des revendications 5 à 6, **caractérisé en ce que** les extrémités libres des lamelles (24) présentent une surface d'extrémité (34) à courbure convexe et **en ce que** les surfaces d'extrémité (34) de toutes les lamelles (24) se trouvent dans une surface enveloppante de forme sphérique.

8. Gratte-langue selon l'une des revendications 5 à 7, **caractérisé en ce qu'**au moins l'une des parois avant ou arrière (40 ; 38) des lamelles (24) est rectiligne.

9. Gratte-langue selon l'une des revendications 5 à 8, **caractérisé en ce que** l'épaisseur (S) de la base de la lamelle (26) augmente dans le sens d'extension du raccord de tube (6) vers le centre.

10. Gratte-langue selon l'une des revendications 5 à 9, **caractérisé en ce qu'il** est prévu entre une face arrière d'une lamelle (24) et une base de lamelle (26) associée prévue à cette fin à proximité de la lamelle (24) au moins une ouverture d'évacuation (30) communiquant avec le canal d'évacuation (8) et **en ce qu'**il est prévu entre l'une des lamelles (24) et une base de lamelle (26) associée à la lamelle (24) prévue à cette fin à proximité, un canal de drainage (28) menant à l'ouverture d'évacuation (30).

11. Gratte-langue selon la revendication 10, **caractérisé en ce que** le canal de drainage (28) est conçu pour être incliné dans le sens de l'ouverture d'évacuation (30) et **en ce que** le canal de drainage (28) se termine du côté bord en dessous d'une bordure (20) entourant la surface avant (18) côté utilisation, qui entoure la surface avant (18) du côté utilisation et qui est prévue à fleur d'une lamelle (24) et/ou d'une base de lamelle (26) .

12. Gratte-langue selon l'une des revendications 5 à 11, **caractérisé en ce que** des ouvertures d'évacuation (30) sont prévues dans chaque cas dans le sens d'extension du raccord de tube (6) à l'avant et à l'arrière des lamelles (24) .

13. Gratte-langue selon l'une des revendications précédentes, **caractérisé en ce que** le corps (2) est sensiblement de forme plate et dépasse au niveau de la face arrière (17) uniquement par l'embout cylindrique (4) et la section de tube conique (10).

14. Gratte-langue selon l'une des revendications précédentes, **caractérisé par** un corps de base (44) qui est pourvu d'un surmoulage, à base d'un composant mou, formant au moins le profilé.

15. Gratte-langue selon l'une des revendications précédentes, **caractérisé en ce que** le surmoulage forme le profilé ainsi qu'un coussin (48) formé sur la face arrière du gratte-langue et **en ce que** le coussin (48) est formé d'un autre composant en matière plastique, en particulier d'un autre composant mou, comme le profilé (22).

16. Gratte-langue selon l'une des revendications précédentes, **caractérisé en ce qu'**un coussin (48) pourvu de picots (52, 54) est prévu sur la face arrière (17) du corps en forme de disque (2) et **en ce que** le coussin (48) est formé d'un composant mou.
